# EUROPÄISCHE PATENTSCHRIFT

(11) **EP 4 168 086 B1**
(45) Veröffentlichungstag und Bekanntmachung des Hinweises auf die Patenterteilung: **27.03.2024**
(21) Anmeldenummer: 22702857.8
(22) Anmeldetag: 20.01.2022
(51) Int. Cl.: A61M 16/06

(54) **MASKENANORDNUNG**
MASK ASSEMBLY
ENSEMBLE MASQUE

(30) Priorität: 02.02.2021 DE 102021102308
(43) Veröffentlichungstag der Anmeldung: 26.04.2023
(73) Patentinhaber: Drägerwerk AG & Co. KGaA, 23558 Lübeck (DE)
(72) Erfinder: HANSMANN, Hans-Ullrich, 23558 Lübeck (DE)
(86) Internationale Anmeldenummer: PCT/DE2022/100057
(87) Internationale Veröffentlichungsnummer: WO 2022/167036

(56) Entgegenhaltungen:
- EP-A1- 3 858 410
- WO-A1-2015/145390
- WO-A1-2016/159787
- WO-A1-2018/097470
- US-A- 4 328 797
- US-A1- 2003 047 189
- US-A1- 2003 168 063
- US-A1- 2008 230 072
- US-A1- 2012 055 480
- US-A1- 2012 125 338

## Beschreibung

Die vorliegende Erfindung betrifft eine Maskenanordnung für ein Beatmungsgerät zum Beatmen eines Patienten.

Im Stand der Technik sind unterschiedliche Beatmungsgeräte mit unterschiedlichen Atemmasken für sogenannte High-Flow-Therapien bekannt. Die bekannten Geräte werden in der Regel relativ einfach gehalten und arbeiten direkt mit einer Sauerstoffquelle zusammen. Reste von Ausatemluft des letzten Atemzuges eines Patienten werden hierbei ausgespült und der nächste Atemzug beginnt, ohne dass das neue Tidalvolumen Reste des letzten Atemzuges enthält. Damit wird der Anteil an kohlendioxidhaltiger, rückgeatmeter Luft stark vermindert. Diese Technik hilft Patienten, die eigenständig und ausreichend Atemarbeit aufbringen können. Eine Druckunterstützung durch das High-Flow-Gerät findet nicht statt. Die High-Flow-Therapie wird häufig für Patienten eingesetzt, die unter der Effizienz eines pulmolaren Stoffaustausches leiden. Ein typisches Gerät ist hierfür ein klinischer Blender oder ein Sauerstoff-Flowmeter mit Nasenkanülen.

Weiterhin gibt es sogenannte CPAP Geräte, die einen konstanten Druck bereitstellen, wodurch der Patient auf diesem erhöhten Druckniveau atmen kann. Durch den erhöhten Druck werden die Atemwege offengehalten und die Gasaustauschfläche wird vergrößert. Die Ausatmung erfolgt meist über einfache Ausströmöffnungen. Auch hier findet eine kontinuierliche Spülung statt. Es wird jedoch lediglich der Maskeninnenraum bzw. ein Maskenvolumen zwischen Atemmaske und Patient gespült. Das Maskenvolumen ist additiv und entsteht durch das Hinzufügen der Atemmaske. Typische Krankheitsbilder hierfür sind chronisch obstruktive Lungenerkrankungen (COPD) oder akutes Lungenversagen (ARDS). Die zur Behandlung bekannten Geräte dienen zum Spülen der oberen nasalen Atemwege mit Sauerstoff oder kohlendioxidfreier Raumluft.

Patienten, die mit High-Flow-Geräten therapiert werden, müssen sicher und ausreichend spontan atmen können. Aufgrund der angewendeten Technik der massiven Überströmung ist eine Überwachung der Atemaktivität kaum möglich. Zusätzlich wird ggf. ein hoher Gasverbrauch in Kauf genommen. Neben den Kosten und der Logistik für die Gase wird hier in der Regel auch ein zusätzlicher Anfeuchter benötigt. Es entstehen schnell Reizungen der obersten Atemwege durch die hohe Strömungsgeschwindigkeit. Patienten, die mit CPAP-Geräten therapiert werden, müssen ebenfalls sicher spontan atmen können. Allerdings sind die Volumenströme in der Inspiration und in der Exspiration stark unterschiedlich, so dass maschinenseitig ein Atemmuster erkannt und überwacht werden kann. Eine Spülung der anatomischen Toträume erfolgt nicht.

Gattungsgemäße Maskenanordnungen und Beatmungsgeräte können den Patentdokumenten WO 2016/159787 A1, US 2012/055480 A1, US 2012/125338 A1 und WO 2015/145390 A1 entnommen werden.

Aufgabe der vorliegenden Erfindung ist es, der voranstehend beschriebenen Problematik zumindest teilweise Rechnung zu tragen. Insbesondere ist es Aufgabe der vorliegenden Erfindung, ein System zum Beatmen von Patienten zur Verfügung zu stellen, die ein angehobenes Druckniveau und gleichzeitig einen funktionalen Stoffaustausch benötigen, während der Volumenstrom möglichst geringgehalten wird und ein Atemmuster erkannt und/oder überwacht werden kann.

Die voranstehende Aufgabe wird durch die Patentansprüche gelöst. Insbesondere wird die voranstehende Aufgabe durch die Maskenanordnung gemäß Anspruch 1 gelöst. Weitere Vorteile der Erfindung ergeben sich aus den Unteransprüchen, der Beschreibung und den Figuren. Dabei gelten Merkmale, die im Zusammenhang mit der Maskenanordnung beschrieben sind auch im Zusammenhang mit dem erfindungsgemäßen Haltemittel, dem erfindungsgemäßen Beatmungsgerät und jeweils umgekehrt, sodass bezüglich der Offenbarung zu den einzelnen Erfindungsaspekten stets wechselseitig Bezug genommen wird und/oder werden kann.

Gemäß einem ersten Aspekt der vorliegenden Erfindung wird eine Maskenanordnung für ein Beatmungsgerät zum Beatmen eines Patienten zur Verfügung gestellt. Die Maskenanordnung weist eine Atemmaske zum Positionieren über dem Mund und über der Nase des Patienten, eine Nasenkanülenanordnung zum Leiten eines Beatmungsgases in die Nase des Patienten, und ein Haltemittel zum Halten der Nasenkanülenanordnung an der Atemmaske, auf. Das Haltemittel kann ein Befestigungsmittel aufweisen, das zwischen einem ersten Funktionszustand zum Fixieren des Schlauchs in einer festen Halteposition an der Atemmaske und einem zweiten Funktionszustand zum Freigeben des Schlauchs aus der festen Halteposition, für eine Verschiebebewegung zwischen dem Schlauch und der Atemmaske, verstellbar ist

Das heißt, das Befestigungsmittel kann in wenigstens zwei unterschiedliche Funktionszustände verstellt werden, um einerseits das Verschieben des Schlauchs an der Atemmaske zu ermöglichen und andererseits den Schlauch an der Atemmaske zu fixieren, sobald sich der Schlauch in der gewünschten Position an der Atemmaske befindet bzw. sobald sich das Schlauchende und/oder der Prong an der gewünschten Position an oder in der Nase des Patienten befinden. Das Befestigungsmittel ist zum Fixieren von wenigstens einem Teil des Schlauchs in und/oder an der Atemmaske bzw. in einer festen Position relativ zur Atemmaske und/oder zur Maskenöffnung ausgestaltet. Das heißt, der Schlauch muss nicht direkt an der Atemmaske befestigt bzw. an dieser angrenzend befestigt werden oder sein. Vielmehr kann das Befestigungsmittel als Bindeglied für die Befestigung zwischen dem Schlauch und der Atemmaske betrachtet werden.

Die erfindungsgemäße Lösung ist geräteseitig ähnlich einem CPAP-Gerät aufgebaut. Die Gaszufuhr erfolgt jedoch nicht in den Innenraum der Atemmaske, sondern direkt in die Nasenkanülenanordnung und von dort in die Nase des Patienten oder zumindest bis kurz vor die Nase des Patienten. Durch die zusätzliche Atemmaske kann ein gewünschtes Druckniveau hergestellt werden. Demnach wird eine Atemmaske vorgeschlagen, die eine Gaszufuhr auch in den Maskeninnenraum bzw. ein Maskenvolumen ermöglicht, hauptsächlich aber die Gaszufuhr über die Nase des Patienten führt. Durch den Mund sowie die Undichtigkeit des sogenannten Prongs der Nasenkanülenanordnung, kann das Beatmungsgas aus dem nasalen Raum wieder entweichen. Allerdings kann das überschüssige Gas im Maskenvolumen nun unter einem einstellbaren Druck gehalten werden. Der Druck kann mittels einer Überströmeinrichtung oder mittels eines aktiv ansteuerbaren Ausatemventils der Maskenanordnung eingestellt werden. Somit kann der Gasfluss für die Spülung der oberen nasalen Atemwege dienen. Außerdem kann der Gasfluss für eine konstante (CPAP) und eine wechselnde (BIPAP) Druckunterstützung eingesetzt werden.

Insbesondere in Verbindung mit einem aktiven Ausatemventil der Maskenanordnung kann ein Beatmungsmodus eingestellt werden, der einen kontinuierlichen Spülfluss aufrechterhält. Der Beatmungsdruck kann dann so eingestellt werden, dass während der Inspiration ein festgelegter Inspirationsdruck erreicht wird. Ein Exspirationsventil des Beatmungsgerätes ist hierbei überwiegend geschlossen, solange der gewünschte Inspirationsdruck nicht wesentlich überschritten wird.

Während der Exspiration kann das Exspirationsventil zum Einstellen des zu haltenden PEEP (Positive End Expiratory Pressure) geregelt werden. Gleichzeitig wird ein Inspirationsventil des Beatmungsgerätes so weit geöffnet, das frisches Beatmungsgas in Richtung des Patienten fließt. Insbesondere zum Ende der Exspiration sorgt dieses Beatmungsgas für eine Spülung der nasalen Toträume. Vor dem Beginn der nächsten Inspiration befindet sich nun frisches, kohlendioxidfreies Beatmungsgas in den nasalen Toträumen.

Die Nasenkanülenanordnung weist einen Schlauch zum Leiten des Beatmungsgases durch eine Öffnung in der Atemmaske oder vorbei an der Atemmaske hin zum Patienten auf. Am Ende des Schlauchs weist die Nasenkanülenanordnung vorzugsweise einen Prong bzw. ein entsprechendes Endstück bzw. einen Gasauslassabschnitt zum Leiten des Beatmungsgases in die Nase des Patienten auf. Das Haltemittel ist vorzugsweise zum Fixieren des Schlauchs an der Atemmaske konfiguriert und ausgestaltet. Darunter, dass die Atemmaske zum Positionieren über dem Mund und der Nase des Patienten ausgestaltet ist soll insbesondere verstanden werden, dass die Atemmaske zum möglichst fluiddichten Bedecken des Mund-Nasen-Bereichs des Patienten ausgestaltet ist, sodass sich der Mund und die Nase im Maskenvolumen befinden bzw. von der Atemmaske bedeckt bzw. umschlossen sind. Durch die Atemmaske kann eine Dichtwirkung zwischen der Umgebung der Atemmaske und dem Maskenvolumen geschaffen werden. Das heißt, das Maskenvolumen wird durch die Atemmaske gegenüber der Umgebung der Atemmaske möglichst stark abgedichtet. Dadurch kann ein relativ hohes Gesamtdruckniveau für die gewünschte Beatmung des Patienten erreicht werden. Durch den an bzw. in der Nase positionierten Gasauslassabschnitt kann eine Dichtwirkung zwischen dem Maskenvolumen und einem Naseninnenvolumen geschaffen werden. Die Dichtwirkung zwischen dem Maskenvolumen und dem Naseninnenvolumen bzw. einem daran anschließenden Rachenraum durch den Gasauslassabschnitt ist jedoch bevorzugt geringer als die Dichtwirkung zwischen dem Maskenvolumen und der Umgebung der Atemmaske. Vorzugsweise kann der Gasauslassabschnitt dahingehend ausgestaltet sein, dass eine gewisse Leckage im Bereich des Gasauslassabschnitts zwischen dem Naseninnenvolumen und dem Maskenvolumen bewusst ermöglicht wird. Dies kann einen vorteilhaften Effekt hinsichtlich einer Spülung oberer Atemwege und/oder des Rachenraums bewirken.

Gemäß einer weiteren Ausführungsform der vorliegenden Erfindung ist es möglich, dass bei einer Maskenanordnung die Atemmaske eine Maskenwandung mit einer Durchgangsöffnung aufweist und die Nasenkanülenanordnung einen Schlauch aufweist, der sich zum Leiten des Beatmungsgases zum Patienten durch die Durchgangsöffnung erstreckt. Damit kann die Maskenanordnung kompakt zur Verfügung gestellt werden. Durch das Hindurchführen des Schlauchs durch die Atemmaske bzw. durch die Maskenwandung kann die gewünschte Fluiddichtigkeit der Maskenanordnung, beispielsweise im Vergleich zu einem an der Atemmaske vorbeigeführten Schlauch, relativ zuverlässig erreicht werden. Der Schlauch erstreckt sich in einer solchen Ausführungsform demnach von einem Bereich außerhalb der Atemmaske durch die Maskenwandung hindurch Richtung Nase des die Maskenanordnung verwendenden Patienten.

Weiter kann es von Vorteil sein, wenn das Befestigungsmittel bei einer erfindungsgemäßen Maskenanordnung beim Fixieren des Schlauchs in der Halteposition einen Durchgangsbereich zwischen einer Schlauchaußenseite des Schlauchs und einer Innenumfangsfläche der Durchgangsöffnung verschließt. Das Befestigungsmittel kann mithin als Dichtmittel zum fluiddichten Verschließen der Durchgangsöffnung bzw. zum Abdichten des Maskenvolumens gegenüber der Umgebung der Maskenanordnung dienen.

Bei einer erfindungsgemäßen Maskenanordnung ist es zudem möglich, dass wenigstens ein Teil des Befestigungsmittels an der Maskenwandung und/oder am Schlauch befestigt ist. Damit kann auf einfache und platzsparende Weise ein stabiler Bauteilverbund zwischen der Atemmaske und dem Schlauch hergestellt werden.

Gemäß einer weiteren Ausgestaltungsvariante der vorliegenden Erfindung ist es möglich, dass bei einer Maskenanordnung das Befestigungsmittel an der Maskenwandung eine erste Durchgangshülse mit einem sich zum Patienten, der die Atemmaske trägt, hin konisch verjüngenden Innengewinde und am Schlauch eine mantelförmig um einen Teil des Schlauchs herum angeordnete Schraube mit einem konischen Außengewinde zum Verschrauben mit dem konischen Innengewinde der ersten Durchgangshülse und dadurch zum Fixieren des Schlauchs in der Halteposition aufweist. Dies schafft auf einfache und platzsparende Weise ein effektives Befestigungsmittel zum flexiblen Befestigen des Schlauchs an der Atemmaske bzw. in der Durchgangsöffnung. Die Schraube kann durch ein Verschrauben mit der Durchgangshülse elastisch verformt werden, sodass sich die Schraube durch das Verschrauben mit der Durchgangshülse am Schlauch festsetzt. Die Elastizität kann die Schraube durch entsprechende Materialeigenschaften, eine geeignete Dimensionierung und/oder eine sich über die gesamte Länge der Schraube erstreckende Lücke in der mantelförmigen Schraubenwandung zum Verringern des Schraubendurchmessers während des Verschraubens aufweisen.

Ferner ist es möglich, dass bei einer Maskenanordnung das Befestigungsmittel an der Maskenwandung eine mantelförmig um einen Teil des Schlauchs herum ausgestaltete, elastisch verformbare zweite Durchgangshülse mit einem Außengewinde, insbesondere einem zumindest teilweise konischen Außengewinde, und eine Mutter mit einem konischen Innengewinde zum Verschrauben mit dem Außengewinde der zweiten Durchgangshülse und dadurch zum Fixieren des Schlauchs in der Halteposition aufweist.

Darüber hinaus ist es bei einer erfindungsgemäßen Maskenanordnung möglich, dass in einer Radialrichtung betrachtet zwischen einer Schlauchaußenseite und einer Innenumfangsfläche der zweiten Durchgangshülse eine elastisch verformbare Klemmhülse mantelförmig um einen Teil des Schlauchs herum ausgestaltet ist, wobei das Material der Klemmhülse eine höhere Festigkeit als das Material des Schlauchs aufweist. Damit kann der Schlauch vor Beschädigung durch die Klemmhülse geschützt werden. Außerdem kann dadurch eine bessere Kraftverteilung auf den Schlauch erreicht werden. Die Klemmhülse und/oder das Material der Klemmhülse weisen vorzugsweise eine höhere Festigkeit als der Schlauch, als das Schlauchmaterial und/oder als der Schlauch in dem Bereich, in welchem die Klemmhülse mantelförmig um den Schlauch herum ausgestaltet ist, auf. Die Klemmhülse kann im Freigabezustand lose und/oder verschiebbar am Schlauch positioniert sein. Die Gewinde sind in den vorstehend beschriebenen Ausführungsformen jeweils für eine Verschraubung außerhalb der Atemmaske bzw. außerhalb des Maskenvolumens ausgestaltet. Damit kann eine gute Bedienbarkeit des Befestigungsmittels gewährleistet werden.

Weiterhin ist es möglich, dass bei einer Maskenanordnung gemäß der vorliegenden Erfindung das Befestigungsmittel eine an der Maskenwandung angebrachte, elastisch verformbare Membran-Saughülse mit einem Membraninnenvolumen aufweist und die Membran-Saughülse im Bereich der Durchgangsöffnung mantelförmig um den Schlauch herum ausgestaltet ist, wobei die Membran-Saughülse einen Druckanschluss zum Erzeugen eines Unterdrucks im Membraninnenvolumen aufweist. Auch damit lässt sich auf wirkungsvolle Weise die gewünschte Funktionsweise des Befestigungsmittels erreichen. Die Membran-Saughülse ist vorzugsweise an der Atemmaske bzw. an der Maskenwandung im Bereich der Durchgangsöffnung befestig. Zur Erzeugung des Unterdrucks kann eine Pumpe am Druckanschluss angeschlossen werden. Der Unterdruck kann demnach mittels des Druckanschlusses, also nicht allein durch den Druckanschluss, erzeugt werden. Über den Druckanschluss kann Luft bzw. ein Gas, das sich im Membraninnenvolumen befindet, aus dem Membraninnenvolumen gesaugt werden. Dadurch verringert sich das Membraninnenvolumen und die Membran-Saughülse hält den Schlauch fest in der Durchgangsöffnung. Die Membran-Saughülse kann im Bereich der Durchgangsöffnung ringförmig um den Schlauch herum ausgestaltet sein. Die Membran-Saughülse kann ferner form-, kraft- und/oder stoffschlüssig an der Maskenwandung befestigt sein. Bei einer solchen Maskenanordnung ist es ferner möglich, dass im Membraninnenvolumen ein elastisch und/oder plastisch verformbarer Feststoff angeordnet ist. Damit kann der Schlauch besonders stabil in der gewünschten Position fixiert werden.

Zudem kann bei einer Maskenanordnung gemäß der vorliegenden Erfindung das Befestigungsmittel im Bereich der Durchgangsöffnung eine mantelförmig und/oder ringförmig um den Schlauch herum angebrachte, elastisch verformbare Membran-Druckhülse mit einem Membraninnenvolumen aufweisen, wobei die Membran-Druckhülse einen Druckanschluss zum Erzeugen eines Überdrucks im Membraninnenvolumen aufweist. So können die Membran-Druckhülse auf dem Schlauch in die Durchgangsöffnung geschoben, der Schlauch in die gewünschte Position bewegt und anschließend die Membran-Druckhülse aufgeblasen werden, sodass sich diese zwischen dem Schlauch und der Maskenwandung in der Durchgangsöffnung festsetzt und damit den Schlauch in der gewünschten Position an der Atemmaske hält.

Bei einer weiteren Ausführungsform der Maskenanordnung kann der Schlauch im Bereich der Durchgangsöffnung eine dünnere Schlauchwandung als in einem Bereich außerhalb der Durchgangsöffnung aufweisen. Alternativ oder zusätzlich kann der Schlauch bei einer erfindungsgemäßen Maskenanordnung im Bereich der Durchgangsöffnung eine elastisch verformbare Schlauchwandung und/oder eine Schlauchwandung mit einer höheren Elastizität als in einem Bereich außerhalb der Durchgangsöffnung aufweisen. Sobald Beatmungsgas durch den Schlauch geleitet wird, kann sich der Schlauch im Bereich der dünneren und/oder elastischen bzw. elastischeren Schlauchwandung durch den entstehenden Überdruck im Schlauch aufweiten bzw. in Radialrichtung vergrößern und den Schlauch in der Durchgangsöffnung fixieren. Der Bereich der dünneren und/oder flexibleren Schlauchwandung erstreckt sich vorzugsweise über ein Vielfaches der Wandstärke der Maskenwandung, sodass der Schlauch ausreichend flexibel in der gewünschten Position fixiert werden kann. Die dünnere und/oder flexiblere Schlauchwandung erstreckt sich vorzugsweise über eine Länge in einem Bereich zwischen 5 mm und 30 mm. Die dünnere und/oder flexiblere Schlauchwandung kann als monolithischer Bestandteil des Schlauchs ausgestaltet sein.

Gemäß einer weiteren Ausgestaltungsvariante der vorliegenden Erfindung ist es möglich, dass bei einer Maskenanordnung das Befestigungsmittel einen mantelförmig um den Schlauch herum ausgestalteten und im Bereich der Durchgangsöffnung an der Maskenwandung angebrachten, thermoplastisch verformbaren Verbindungsmantel aufweist. Das thermoplastische Material des Befestigungsmittels ist dahingehend konfiguriert, dass es bei gewöhnlicher Umgebungstemperatur relativ fest ist, sodass sich das Befestigungsmittel in einem Haltezustand zum Fixieren des Schlauchs befindet. Soll der Schlauch für die Verschiebebewegung aus der Halteposition freigegeben werden, muss der Verbindungsmantel lediglich erwärmt werden.

Bei den vorstehend beschriebenen Ausführungsformen kann es weiterhin von Vorteil sein, wenn die Maskenanordnung ein Verbindungsgelenk zum angelenkten Halten des Schlauchs in der Durchgangsöffnung aufweist. Damit kann sichergestellt werden, dass der Schlauch nicht vollständig oder zu weit aus der Durchgangsöffnung rutscht, sobald der Schlauch nicht durch das Befestigungsmittel an und/oder in der Atemmaske befestigt ist. Durch das Verbindungsgelenk lässt sich der Schlauch im Bereich der Durchgangsöffnung für die gewünschte Positionierung jedoch trotzdem noch verschieben. Das Verbindungsgelenk kann innerhalb und/oder außerhalb der Durchgangsöffnung an der Atemmaske sowie am Schlauch ausgestaltet sein.

Bei einer erfindungsgemäßen Maskenanordnung kann ein Teil des Schlauchs, der sich bei der Verwendung der Maskenanordnung in einem durch die Atemmaske am Patienten gebildeten Maskenvolumen befindet, plastisch verformbar sein. Auch damit lässt sich die Position des Schlauchendes bzw. die Position eines Gasauslassabschnitts an und/oder in der Nase flexibel an den jeweiligen Patienten anpassen bzw. in die gewünschte Position bringen.

Außerdem ist es möglich, dass die Nasenkanülenanordnung bei einer erfindungsgemäßen Maskenanordnung einen Gasauslass zum Auslassen des Beatmungsgases in die Nase des Patienten aufweist, wobei der Gasauslass düsenförmig ausgestaltet ist. Damit kann ein zielgerichteter Gasauslass erzeugt werden, der das Beatmungsgas über den Gasauslass hinaus in die gewünschte Richtung leiten kann. Damit ist es möglich, die Nasenkanülen bzw. den Prong nicht in die Nase des Patienten, sondern kurz bzw. knapp vor der Nase des Patienten zu positionieren. Dies trägt zu einem verbesserten Tragekomfort und folglich zu einer höheren Akzeptanz der Maskenanordnung bei.

Von weiterem Vorteil kann es sein, wenn die Nasenkanülenanordnung einer erfindungsgemäßen Maskenanordnung einen Gasauslassabschnitt zum Auslassen des Beatmungsgases in die Nase des Patienten aufweist, wobei sich der Gasauslassabschnitt in einer Beatmungsgasleitungsrichtung konisch verjüngt. Damit lässt sich der Gasauslassabschnitt der Nasenkanülenanordnung einfach und sicher in der Nase des Patienten positionieren.

Weiterhin kann die Nasenkanülenanordnung einer erfindungsgemäßen Maskenanordnung einen Gasauslassabschnitt zum Auslassen des Beatmungsgases in die Nase des Patienten aufweisen, wobei im und/oder am Gasauslassabschnitt eine Ballondichtung mit einem Ballonmantel ausgestaltet ist und wobei der Ballonmantel eine Gasöffnung zum Zuführen des Beatmungsgases in der Nasenkanülenanordnung bei einer Beatmung des Patienten in ein durch den Ballonmantel gebildetes Ballonvolumen zum druckbeaufschlagten Halten des Gasauslassabschnitts in der Nase aufweist. Damit lässt sich eine stabile Positionierung des Gasauslassabschnitts in der Nase des Patienten erreichen.

Darüber hinaus kann die Nasenkanülenanordnung einer Maskenanordnung einen Gasauslassabschnitt zum Auslassen des Beatmungsgases in die Nase des Patienten aufweisen, wobei die Maskenanordnung ferner eine aufblasbare Schlauchdichtung aufweist, die ringförmig um den Gasauslassabschnitt herum ausgestaltet ist. Auch damit lässt sich der Gasauslassabschnitt stabil bzw. bewegungshemmend in der Nase des Patienten positionieren. Die Schlauchdichtung kann einen Druckanschluss aufweisen, über welchen die Schlauchdichtung mittels Pumpe aufblasbar und somit in der Nase des Patienten fixierbar ist. Eine Druckluftleitung zum Aufblasen der Schlauchdichtung kann außerhalb der Atemmaske in den Schlauch geführt werden bzw. sein und sich dort bis hin zur Schlauchdichtung und/oder zum vorstehend erwähnten Druckanschluss erstrecken.

Gemäß einer weiteren Ausführungsform der vorliegenden Erfindung ist es möglich, dass die Nasenkanülenanordnung einen Gasauslassabschnitt zum Auslassen des Beatmungsgases in die Nase des Patienten aufweist, wobei der Gasauslassabschnitt wenigstens einen elastisch verformbaren Federarm, zum druckbeaufschlagten Halten des Gasauslassabschnitts in der Nase, aufweist. Der wenigstens eine Federarm ist eine einfache und trotzdem zuverlässige Möglichkeit einer stabilen Positionierung des Gasauslassabschnitts in der Nase des Patienten. Der wenigstens eine Federarm kann zudem dahingehend ausgestaltet sein, dass weiterhin Gas aus der Nase des Patienten strömen kann. Der Gasauslassabschnitt weist vorzugsweise wenigstens zwei oder vier Federarme auf. Anstelle der Federarme können am Gasauslassabschnitt auch Federtrichter ausgestaltet sein. Diese führen zu einer noch stabileren Positionierung des Gasauslassabschnitts in der Nase des Patienten. Außerdem verhindern diese zumindest weitestgehend einen Gasauslass aus der Nase des Patienten.

Von weiterem Vorteil kann es sein, wenn eine Maskenanordnung gemäß der vorliegenden Erfindung an der Atemmaske eine Haltebandanordnung mit einem Stirnband und/oder einem Nackenband zum Halten der Atemmaske am Patienten aufweist. Damit kann nicht nur die Atemmaske, sondern auch die Nasenkanülenanordnung stabil in der gewünschten Position gehalten werden. Weiterhin können das Stirnband und/oder das Nackenband verstellbar, zum Anpassen der Maskenanordnung an den jeweiligen Patienten, ausgestaltet sein. Durch den Überdruck bei der Verwendung der Maskenanordnung muss die Atemmaske möglichst leckagefrei an das Gesicht des Patienten gedrückt oder gezogen werden. Mit der erfindungsgemäßen Bebänderung können die Leckagen auf Werte kleiner 10 L/min oder kleiner 20 L/min gehalten werden. Unter dem Nackenband kann eine Bebänderung zum Halten der Atemmaske im Bereich der Mundwinkel des Patienten verstanden werden. Unter dem Stirnband kann eine Bebänderung zum Halten der Atemmaske im Bereich des Nasenansatzes des Patienten verstanden werden. Beide Bänder können mittels eines Klettverschlusses in der Länge eingestellt bzw. verstellt werden. Die Bänder sind möglichst wenig elastisch und/oder entsprechend steif bzw. mit einer entsprechend hohen Festigkeit ausgestaltet.

Die Atemmaske einer erfindungsgemäßen Maskenanordnung kann einen Maskenrand zum Anlegen der Atemmaske am Gesicht des Patienten aufweisen, wobei der Maskenrand eine umlaufende, elastisch verformbare Silikonlippe und/oder ein entsprechend geformtes Elastomer aufweist. Damit ist es möglich, eine geringe Leckage aus dem Maskenvolumen in die Umgebung der Maskenanordnung bei gleichzeitig hohem Tragekomfort der Atemmaske zu erreichen.

Weiterhin ist es bei einer Maskenanordnung gemäß der vorliegenden Erfindung möglich, dass die Atemmaske einen elastisch verformbaren Maskenrand zum Anlegen der Atemmaske am Gesicht des Patienten aufweist, wobei der Maskenrand eine Randkammer mit einem Randvolumen aufweist, in welchem zur Umgebung der Maskenanordnung Überdruck herrscht. Auch damit können ein hoher Tragekomfort bei gleichzeitig effektiver Dichtwirkung geschaffen werden.

Für einen hohen Tragekomfort und die gewünschte Dichtwirkung kann die Atemmaske einer erfindungsgemäßen Maskenanordnung außerdem einen gel- und/oder schwammartigen Maskenrand zum Anlegen der Atemmaske am Gesicht des Patienten aufweisen.

Offenbart wird auch ein Haltemittel zum Halten einer Nasenkanülenanordnung an einer Atemmaske in einer wie vorstehend beschriebenen Maskenanordnung. Außerdem wird ein Beatmungsgerät zum Beatmen eines Patienten zur Verfügung offenbart.

Weitere, die Erfindung verbessernde Maßnahmen ergeben sich aus der nachfolgenden Beschreibung zu verschiedenen Ausführungsbeispielen der Erfindung, welche in den Figuren schematisch dargestellt sind. Sämtliche aus den Ansprüchen, der Beschreibung oder den Figuren hervorgehende Merkmale und/oder Vorteile, einschließlich konstruktiver Einzelheiten und räumlicher Anordnungen können sowohl für sich als auch in den verschiedenen Kombinationen erfindungswesentlich sein.

Es zeigen jeweils schematisch:
- Figur 1: ein Beatmungsgerät mit einer Maskenanordnung gemäß einer ersten Ausführungsform der vorliegenden Erfindung,
- Figur 2: Details einer Maskenanordnung gemäß einer zweiten Ausführungsform der vorliegenden Erfindung,
- Figur 3: Details einer Maskenanordnung gemäß einer dritten Ausführungsform der vorliegenden Erfindung in einem ersten Funktionszustand,
- Figur 4: Details einer Maskenanordnung gemäß der dritten Ausführungsform der vorliegenden Erfindung in einem zweiten Funktionszustand,
- Figur 5: Details einer Maskenanordnung gemäß einer vierten Ausführungsform der vorliegenden Erfindung in einem ersten Funktionszustand,
- Figur 6: Details einer Maskenanordnung gemäß der vierten Ausführungsform der vorliegenden Erfindung in einem zweiten Funktionszustand,
- Figur 7: Details einer Maskenanordnung gemäß einer fünften Ausführungsform der vorliegenden Erfindung in einem ersten Funktionszustand,
- Figur 8: Details einer Maskenanordnung gemäß der fünften Ausführungsform der vorliegenden Erfindung in einem zweiten Funktionszustand,
- Figur 9: Details einer Maskenanordnung gemäß einer sechsten Ausführungsform der vorliegenden Erfindung,
- Figur 10: Details einer Maskenanordnung gemäß einer siebten Ausführungsform der vorliegenden Erfindung,
- Figur 11: Details einer Maskenanordnung gemäß einer achten Ausführungsform der vorliegenden Erfindung,
- Figur 12: Details einer Maskenanordnung gemäß einer neunten Ausführungsform der vorliegenden Erfindung in einem ersten Funktionszustand,
- Figur 13: Details einer Maskenanordnung gemäß der neunten Ausführungsform der vorliegenden Erfindung in einem zweiten Funktionszustand,
- Figur 14: Details einer Maskenanordnung gemäß einer zehnten Ausführungsform der vorliegenden Erfindung in einem ersten Funktionszustand,
- Figur 15: Details einer Maskenanordnung gemäß der zehnten Ausführungsform der vorliegenden Erfindung in einem zweiten Funktionszustand,
- Figur 16: Details einer Maskenanordnung gemäß einer elften Ausführungsform der vorliegenden Erfindung,
- Figur 17: Details einer Maskenanordnung gemäß einer zwölften Ausführungsform der vorliegenden Erfindung,
- Figur 18: Details einer Maskenanordnung gemäß einer dreizehnten Ausführungsform der vorliegenden Erfindung,
- Figur 19: Details einer Maskenanordnung gemäß einer vierzehnten Ausführungsform der vorliegenden Erfindung,
- Figur 20: Details einer Maskenanordnung gemäß einer fünfzehnten Ausführungsform der vorliegenden Erfindung,
- Figur 21: Details einer Maskenanordnung gemäß einer sechzehnten Ausführungsform der vorliegenden Erfindung,
- Figur 22: Details einer Maskenanordnung gemäß einer siebzehnten Ausführungsform der vorliegenden Erfindung,
- Figur 23: ein Flussdiagramm zum Erläutern eines Verfahrens.

Elemente mit gleicher Funktion und Wirkungsweise sind in den Figuren jeweils mit den gleichen Bezugszeichen versehen.

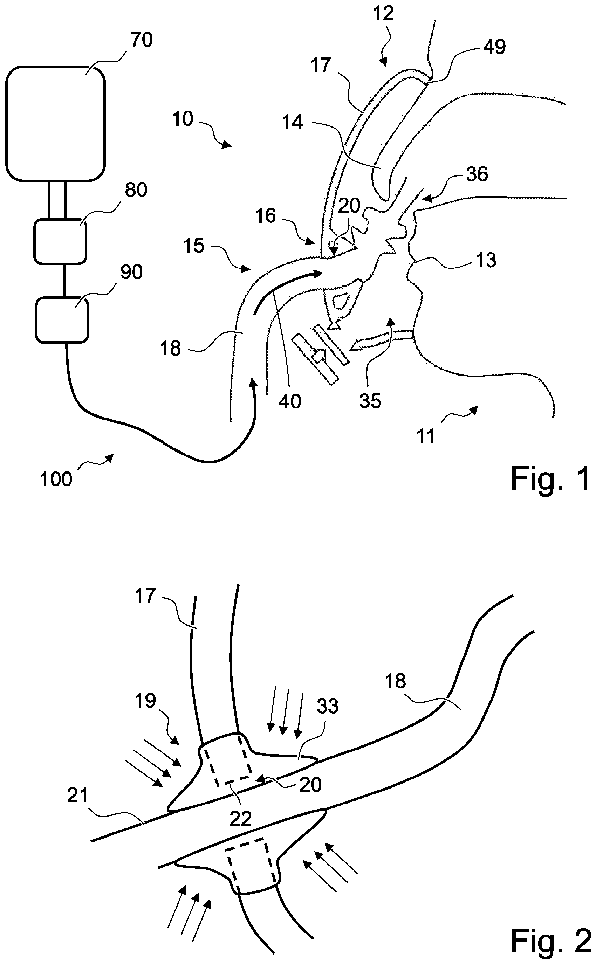

Fig. 1 zeigt ein Beatmungsgerät 100 zum Beatmen eines Patienten 11. Das Beatmungsgerät 100 weist eine Gasquelle 70, einen Blender 80, einen Befeuchter 90 und eine Maskenanordnung 10 auf. Die Gasquelle 70 weist zwei Druckgasanschlüsse für Sauerstoff und für medizinische Luft auf. Im Blender 80 werden die beiden Gasströme zusammengeführt. Von dort wird das eingestellte Gemisch aus Luft und Sauerstoff mit einem möglichst konstanten Volumenstrom über den Befeuchter 90 als Beatmungsgas in einer Beatmungsrichtung 40 zum Patienten 11 geführt.

Die Maskenanordnung 10 umfasst eine Atemmaske 12, die über bzw. auf dem Mund 13 und der Nase 14 des Patienten 11 positioniert ist. Ferner umfasst die Maskenanordnung 10 eine Nasenkanülenanordnung 15 zum Leiten des Beatmungsgases in die Nase 14 des Patienten 11 und ein Haltemittel 16 zum Halten der Nasenkanülenanordnung 15 an der Atemmaske 12. Die Atemmaske 12 weist eine Maskenwandung 17 mit einer Durchgangsöffnung 20 auf. Die Nasenkanülenanordnung 15 weist einen Schlauch 18 auf, der sich, zum Leiten des Beatmungsgases zum Patienten 11, durch die Durchgangsöffnung 20 erstreckt. Zum Anpassen der Nasenkanülenanordnung 15 an den jeweiligen Patienten 11 ist ein Endabschnitt des Schlauchs 18 im Maskenvolumen 35 plastisch verformbar. Im Bereich der Nase 14 des Patienten 11 weist der Schlauch 18 einen Gasauslassabschnitt 36 auf.

Durch die Atemmaske 12 wird eine Dichtwirkung zwischen der Umgebung der Atemmaske 12 und dem Maskenvolumen 35 geschaffen. Das heißt, das Maskenvolumen 35 wird durch die Atemmaske 12 gegenüber der Umgebung der Atemmaske 12 möglichst stark abgedichtet. Dadurch kann ein relativ hohes Gesamtdruckniveau für die gewünschte Beatmung des Patienten 11 erreicht werden. Durch den an bzw. in der Nase 14 positionierten Gasauslassabschnitt 36 kann eine Dichtwirkung zwischen dem Maskenvolumen 35 und einem Naseninnenvolumen geschaffen werden. Die Dichtwirkung zwischen dem Maskenvolumen 35 und dem Naseninnenvolumen durch den Gasauslassabschnitt 36 ist jedoch geringer als die Dichtwirkung zwischen dem Maskenvolumen 35 und der Umgebung der Atemmaske 12. In der dargestellten Ausführungsform ist der Gasauslassabschnitt 36 deshalb dahingehend ausgestaltet, dass eine gewisse Leckage im Bereich des Gasauslassabschnitts 36 zwischen dem Naseninnenvolumen und dem Maskenvolumen 35 ermöglicht wird.

Fig. 2 zeigt eine Detaillösung einer Maskenanordnung 10 gemäß einer zweiten Ausführungsform. Gemäß der in Fig. 2 dargestellten Ausführungsform umfasst das Haltemittel 16 ein Befestigungsmittel 19 zum Fixieren des Schlauchs 18 in einer Halteposition an der Atemmaske 12 bzw. an und in der Durchgangsöffnung 20 sowie zum Freigeben des Schlauchs 18 aus der Halteposition für eine Verschiebebewegung zwischen dem Schlauch 18 und der Atemmaske 12. Das in Fig. 2 gezeigte Befestigungsmittel 19 verschließt beim Fixieren des Schlauchs 18 in der Halteposition einen Durchgangsbereich zwischen einer Schlauchaußenseite 21 des Schlauchs 18 und einer Innenumfangsfläche 22 der Durchgangsöffnung 20. Das gezeigte Befestigungsmittel 19 weist einen mantelförmig um den Schlauch 18 herum ausgestalteten und im Bereich der Durchgangsöffnung 20 an der Maskenwandung 17 angebrachten, thermoplastisch verformbaren Verbindungsmantel 33 auf. Wird der Verbindungsmantel 33 erwärmt, lässt sich der Schlauch 18 in der Durchgangsöffnung 20 bzw. im Verbindungsmantel 33 bewegen und in die gewünschte Position bringen. Kühlt der Verbindungsmantel 33 wieder ab bzw. nimmt Umgebungstemperatur an, wird der Schlauch 18 in seiner Position fixiert. Der Verbindungsmantel 33 ist im Bereich der Durchgangsöffnung 20 an der Maskenwandung 17 befestigt.

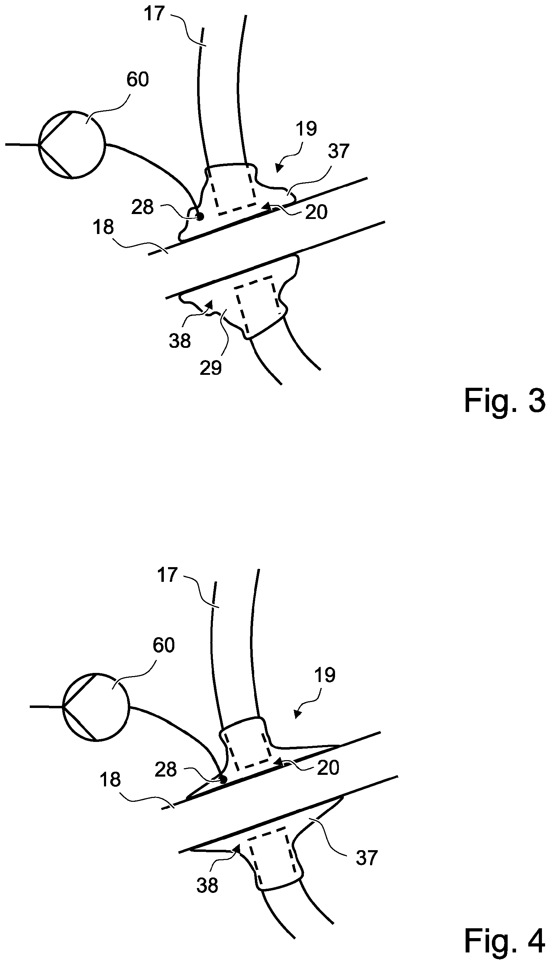

Fig. 3 zeigt eine Detaillösung einer Maskenanordnung 10 gemäß einer dritten Ausführungsform. Gemäß der in Fig. 3 gezeigten Ausführungsform umfasst das Befestigungsmittel 19 eine an der Maskenwandung 17 angebrachte, elastisch verformbare Membran-Saughülse 37 mit einem Membraninnenvolumen 38, wobei die Membran-Saughülse 37 im Bereich der Durchgangsöffnung 20 mantelförmig um den Schlauch 18 herum ausgestaltet ist und wobei die Membran-Saughülse 37 einen Druckanschluss 28 zum Erzeugen eines Unterdrucks im Membraninnenvolumen 38 aufweist. Im Membraninnenvolumen 38 ist ferner ein elastisch verformbarer Feststoff 29 in Form von Styropor angeordnet. Fig. 3 zeigt die Membran-Saughülse 37 in einem Normaldruckzustand. Fig. 4 zeigt die Membran-Saughülse 37 in einem Unterdruckzustand, in welchem in der Membran-Saughülse 37 durch eine Pumpe 60 Unterdruck erzeugt wurde bzw. ist und sich die Membran-Saughülse 37 dadurch am Schlauch 18 festsaugt und diesen damit an der Atemmaske 12 befestigt.

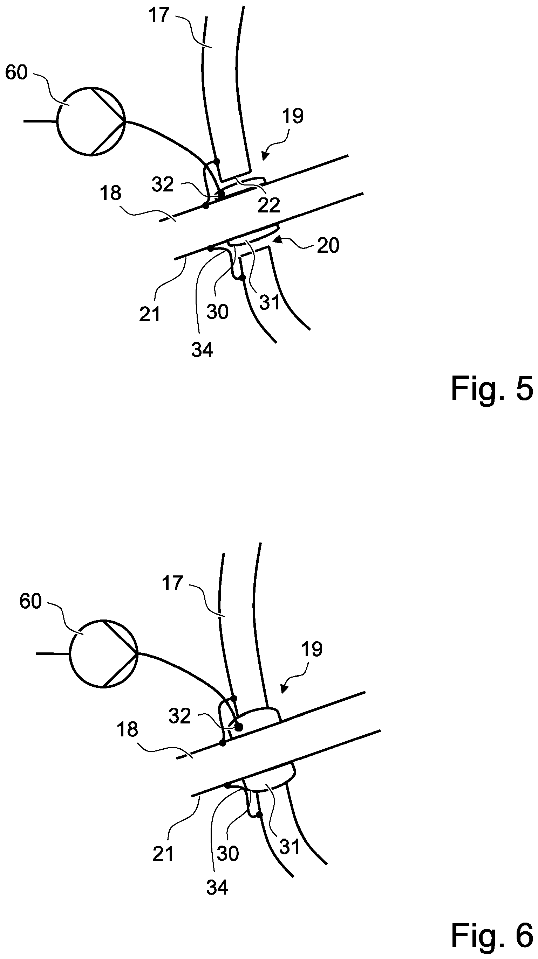

Fig. 5 zeigt eine Detaillösung einer Maskenanordnung 10 gemäß einer vierten Ausführungsform. Gemäß der in Fig. 5 dargestellten Ausführungsform weist das Befestigungsmittel 19 im Bereich der Durchgangsöffnung 20 eine mantelförmig um den Schlauch 18 herum angebrachte, elastisch verformbare Membran-Druckhülse 30 mit einem Membraninnenvolumen 31 auf. Die Membran-Druckhülse 30 weist einen Druckanschluss 32 zum Erzeugen eines Überdrucks im Membraninnenvolumen 30 auf. Ferner weist die in Fig. 5 gezeigte Maskenanordnung 10 ein Verbindungsgelenk 34 in Form von zwei flexiblen Verbindungsbändern zum angelenkten Halten des Schlauchs 18 in der Durchgangsöffnung 20 auf. Fig. 5 zeigt die Membran-Druckhülse 30 in einem Normaldruckzustand, in welchem der Schlauch 18 in der Durchgangsöffnung 20 zur gewünschten Positionierung bewegt bzw. verschoben werden kann. Fig. 6 zeigt die Membran-Druckhülse 30 in einem Überdruckzustand, in welchem in der Membran-Druckhülse 30 durch eine Pumpe 60 Überdruck erzeugt wurde bzw. ist und sich die Membran-Druckhülse 30 dadurch in der Durchgangsöffnung 20 ausbreitet bzw. aufgeblasen wird, bis der Schlauch 18 in der gewünschten Position an der Atemmaske 12 fixiert ist.

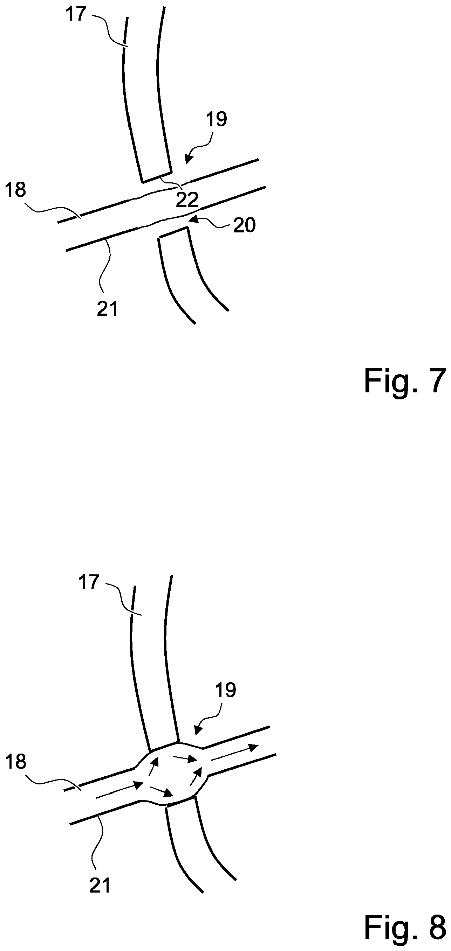

Fig. 7 zeigt eine Detaillösung einer Maskenanordnung 10 gemäß einer fünften Ausführungsform. Gemäß der in Fig. 7 dargestellten Ausführungsform weist der Schlauch 18 im Bereich der Durchgangsöffnung 20 eine dünnere Schlauchwandung als in einem Bereich außerhalb der Durchgangsöffnung 20 auf. Damit weist der Schlauch 18 im Bereich der Durchgangsöffnung 20 eine höhere Elastizität als im Bereich außerhalb der Durchgangsöffnung 20 auf. Wird nun, wie in Fig. 8 dargestellt, Beatmungsgas durch den Schlauch 18 zum Patienten 11 geleitet, dehnt sich die elastische bzw. elastischere Schlauchwandung im Bereich der Durchgangsöffnung 20 durch den erhöhten Gasdruck im Schlauch 18 radial nach außen und fixiert den Schlauch 18 damit in der Durchgangsöffnung 20.

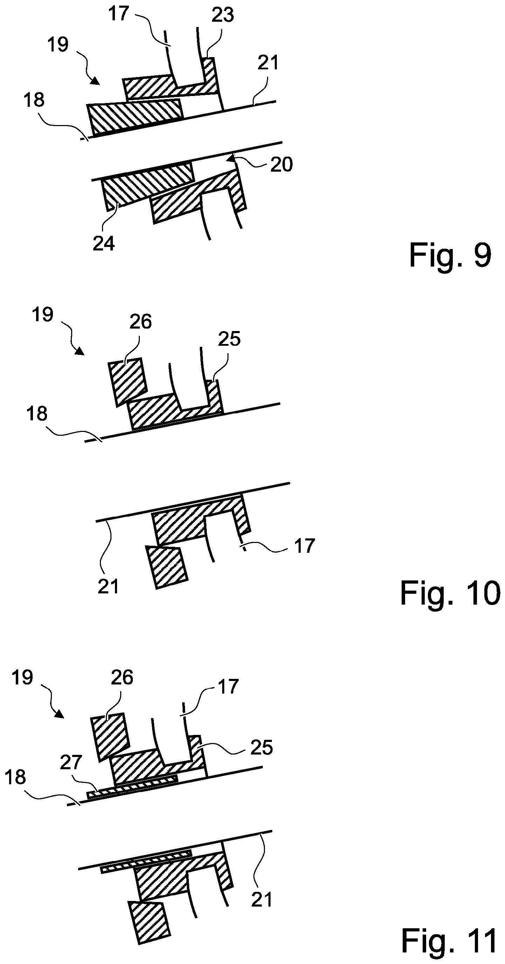

Fig. 9 zeigt eine Detaillösung einer Maskenanordnung 10 gemäß einer sechsten Ausführungsform. Gemäß der in Fig. 9 dargestellten Ausführungsform weist das Befestigungsmittel 19 an der Maskenwandung 17 eine erste Durchgangshülse 23 mit einem sich zum Patienten 11, der die Atemmaske 12 trägt, hin konisch verjüngenden Innengewinde und am Schlauch 18 eine mantelförmig um einen Teil des Schlauchs 18 herum angeordnete Schraube 24 bzw. Schraubenhülse mit einem konischen Außengewinde zum Verschrauben mit dem konischen Innengewinde der ersten Durchgangshülse 23 und dadurch zum Fixieren des Schlauchs 18 in der Halteposition auf.

Gemäß der in Fig. 10 dargestellten siebten Ausführungsform weist das Befestigungsmittel 19 an der Maskenwandung 17 eine mantelförmig um einen Teil des Schlauchs 18 herum ausgestaltete, elastisch verformbare zweite Durchgangshülse 25 mit einem Außengewinde und eine Mutter 26 mit einem konischen Innengewinde zum Verschrauben mit dem Außengewinde der zweiten Durchgangshülse 25 und dadurch zum Fixieren des Schlauchs 18 in der Halteposition auf.

Fig. 11 zeigt eine Detaillösung einer Maskenanordnung 10 gemäß einer achten Ausführungsform, bei welcher in einer Radialrichtung betrachtet zwischen einer Schlauchaußenseite 21 und einer Innenumfangsfläche der zweiten Durchgangshülse 25 eine elastisch verformbare Klemmhülse 27 mantelförmig um einen Teil des Schlauchs 18 herum ausgestaltet ist. Das Material der Klemmhülse 27 weist hierbei eine höhere Festigkeit als das Material des Schlauchs 18 auf.

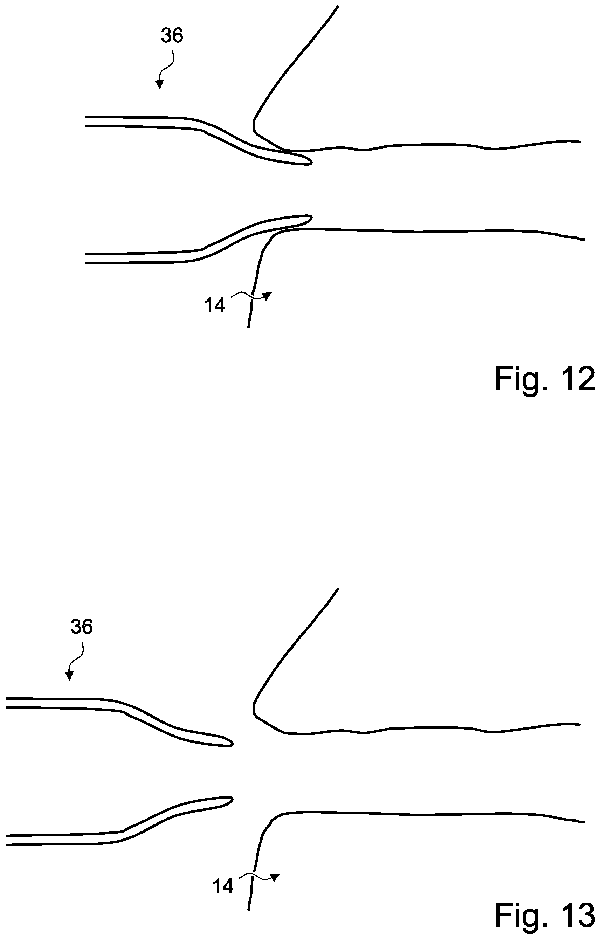

Fig. 12 zeigt eine Detaillösung einer Maskenanordnung 10 gemäß einer neunten Ausführungsform. Hierbei weist die Nasenkanülenanordnung 15 einen düsenförmigen Gasauslass 36 zum Auslassen des Beatmungsgases in die Nase 14 bzw. zur Nase des Patienten 11 auf. Wie in Fig. 13 dargestellt kann der Gasauslass 36 bzw. der Endabschnitt eines Prongs damit ohne oder zumindest ohne starke Abstriche bei der Wirksamkeit der Beatmungsgaszufuhr außerhalb der Nase 14 positioniert werden.

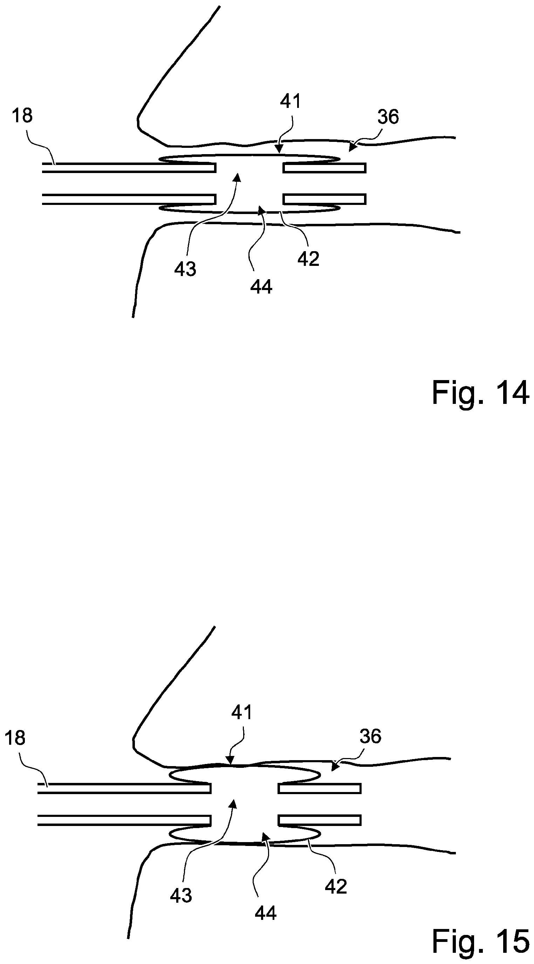

Fig. 14 zeigt eine Detaillösung einer Maskenanordnung 10 gemäß einer zehnten Ausführungsform, bei welcher die Nasenkanülenanordnung 15 einen Gasauslassabschnitt 36 zum Auslassen des Beatmungsgases in die Nase 14 des Patienten 11 aufweist, wobei am Gasauslassabschnitt 36 eine Ballondichtung 41 mit einem Ballonmantel 42 ausgestaltet ist und wobei der Ballonmantel 42 eine Gasöffnung 43 zum Zuführen des Beatmungsgases in der Nasenkanülenanordnung 15 bei einer Beatmung des Patienten 11 in ein durch den Ballonmantel 42 gebildetes Ballonvolumen 44 zum druckbeaufschlagten Halten des Gasauslassabschnitts 36 in der Nase 14 aufweist. Wie in Fig. 15 dargestellt, füllt sich das Ballonvolumen 44 bei der Beatmung des Patienten 11 automatisch mit dem Beatmungsgas und dehnt sich dadurch aus. Der Druck im Ballonvolumen ist gerade stark genug, um den Gasauslassabschnitt 36 stabil in der Nase 14 zu halten und dabei keinen unangenehmen Druck auf die Nasenwand aufzubauen.

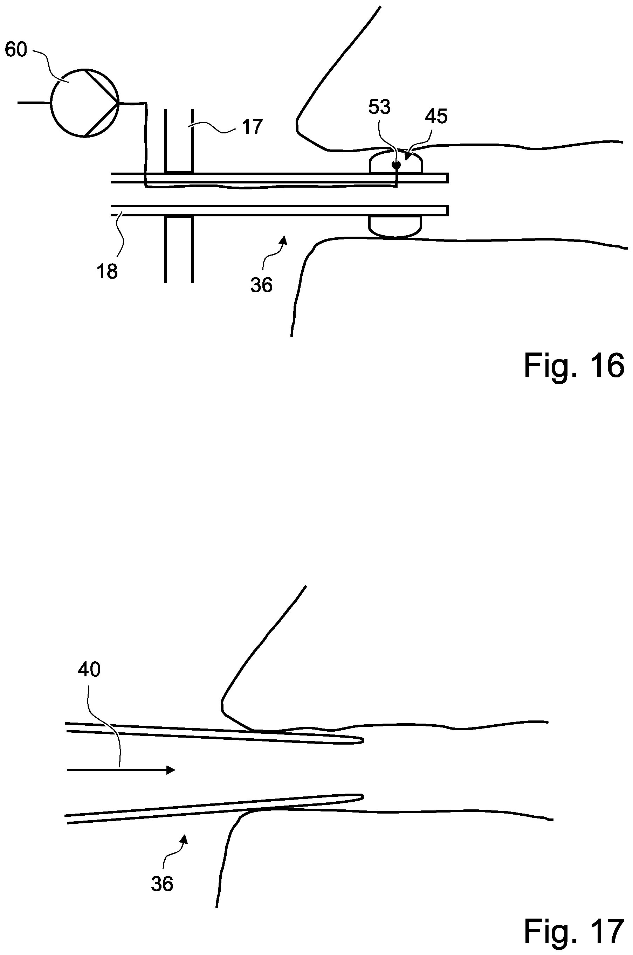

Fig. 16 zeigt eine Detaillösung einer Maskenanordnung 10 gemäß einer elften Ausführungsform, bei welcher die Maskenanordnung 10 eine aufblasbare Schlauchdichtung 45 aufweist, die ringförmig um den Gasauslassabschnitt 36 herum ausgestaltet ist. Die dargestellte Schlauchdichtung 45 weist einen Druckanschluss 53 auf, über welchen die Schlauchdichtung 45 mittels Pumpe 60 aufblasbar und somit in der Nase des Patienten 11 verrutschhemmend fixierbar ist. Eine Druckluftleitung 54 zum Aufblasen der Schlauchdichtung 45 ist außerhalb der Atemmaske 12 in den Schlauch 18 geführt und erstreckt sich dort innerhalb des Schlauchs 18 bis hin zur Schlauchdichtung 45 bzw. zum Druckanschluss 53.

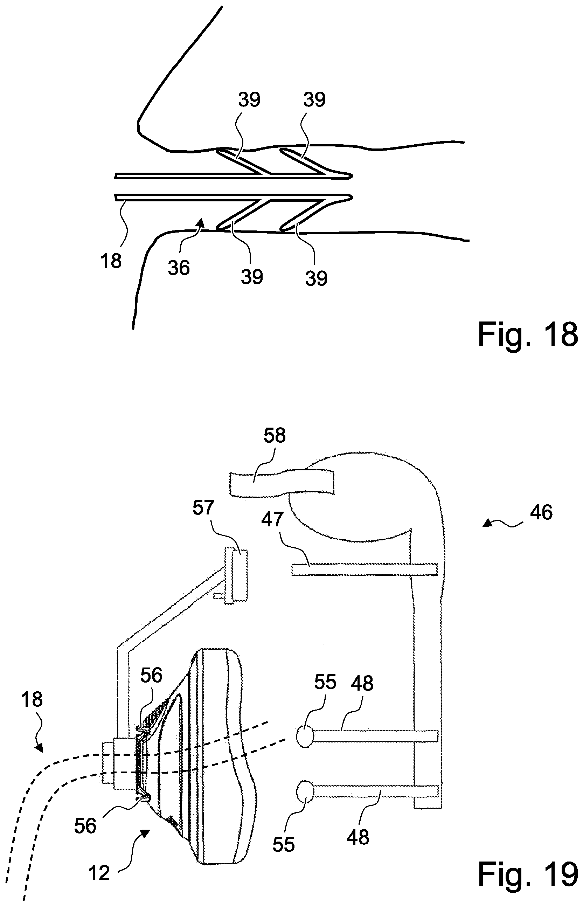

In Fig. 17 ist eine Detaillösung einer Maskenanordnung 10 gemäß einer zwölften Ausführungsform dargestellt, bei welcher der Gasauslassabschnitt 36 in einer Beatmungsgasleitungsrichtung 40 konisch verjüngt ist. Bei der in Fig. 18 gezeigten Detaillösung einer dreizehnten Ausführungsform der Maskenanordnung 10 weist der Gasauslassabschnitt 36 zwei elastisch verformbare Federarme 39, zum druckbeaufschlagten bzw. gespannten Halten des Gasauslassabschnitts 36 in der Nase 14, auf.

Fig. 19 zeigt weitere Detaillösungen für eine Maskenanordnung 10 gemäß einer vierzehnten Ausführungsform. Wie in Fig. 19 dargestellt, weist die Maskenanordnung 10 eine Haltebandanordnung 46 mit einem Stirnband 47, einem oberen Kopfband 58, und zwei Nackenbändern 48 zum Halten der Atemmaske 12 am Patienten 11 auf. An den Nackenbändern 48 sind Halteringe 55 befestigt, die zum Halten der Atemmaske 12 an zugehörigen Haken 56 befestigt werden können. Das Stirnband 47 und das Kopfband 58 können an einer Halteöse 57 befestigt werden. Alternativ zu der dargestellten Bebänderung sind auch Bänder mit Klettverschluss an Stelle der Halteringe 55 und Haken 56 möglich. Auf das Kopfband 58 kann verzichtet werden. Außerdem kann auf eines der Nackenbänder 48 verzichtet werden.

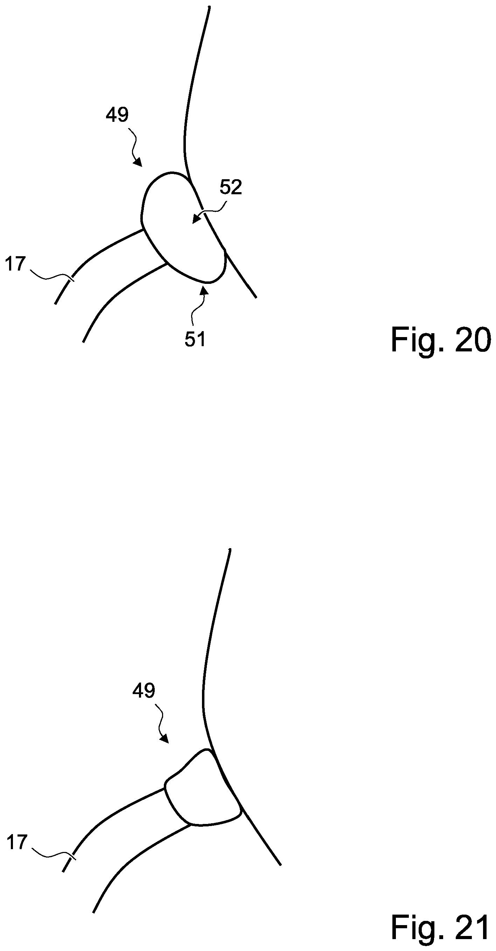

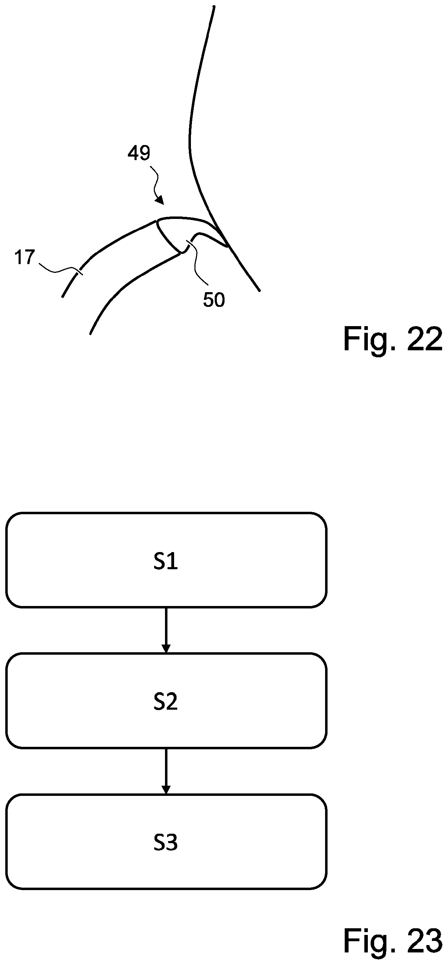

Mit Bezug auf die Figuren 20 bis 22 werden mögliche Ausgestaltungsvarianten eines Maskenrands 49 der Atemmaske 12 beschrieben. Fig. 20 zeigt eine Maskenanordnung 10 gemäß einer fünfzehnten Ausführungsform, bei welcher die Atemmaske 12 einen elastisch verformbaren Maskenrand 49 zum Anlegen der Atemmaske 12 am Gesicht des Patienten 11 aufweist, wobei der Maskenrand 49 eine Randkammer 51 mit einem Randvolumen 52 aufweist, in welchem zur Umgebung der Maskenanordnung 10 Überdruck herrscht. In Fig. 21 ist eine Detaillösung einer sechzehnten Ausführungsform der Maskenanordnung 10 dargestellt, bei welcher die Atemmaske 12 einen gelartigen Maskenrand 49 zum Anlegen der Atemmaske 12 am Gesicht des Patienten 11 aufweist. Der in Fig. 22 dargestellte Maskenrand 49 einer Maskenanordnung 10 gemäß einer siebzehnten Ausführungsform weist eine umlaufende, elastisch verformbare Silikonlippe 50 auf.

Mit Bezug auf Fig. 23 wird anschließend ein Verfahren zum Einstellen einer wie vorstehend dargestellten Maskenanordnung 10 beschrieben. In einem ersten Schritt S1 wird die Atemmaske 12 zunächst über dem Mund 13 und über der Nase 14 des Patienten 11 positioniert. In einem zweiten, insbesondere darauffolgenden Schritt S2 wird der Schlauch 18 relativ zur Atemmaske 12 verschoben, um den Gasauslassabschnitts 36 in oder an der Nase 14 des Patienten 11 in die gewünschte Position zu bringen. In einem dritten Schritt S3 wird der Schlauch 18 durch das Befestigungsmittel 19 in der Halteposition fixiert. Anschließend kann mit der Beatmung des Patienten 11 begonnen werden.

Die Erfindung lässt neben den dargestellten Ausführungsformen weitere Gestaltungsgrundsätze zu. D.h., die Erfindung soll nicht auf die mit Bezug auf die Figuren erläuterten Ausführungsbeispiele beschränkt betrachtet werden. In den Figuren 12 bis 18 sind jeweils nur ein Nasenloch und nur ein Gasauslassabschnitt 36 dargestellt. Die gezeigten Gasauslassabschnitte 36 sind bei einer erfindungsgemäßen Maskenanordnung 10 aber in der Regel doppelt, beispielsweise an einem entsprechend ausgestalteten Prong, vorhanden.

### Bezugszeichenliste

- 10: Maskenanordnung
- 11: Patient
- 12: Atemmaske
- 13: Mund
- 14: Nase
- 15: Nasenkanülenanordnung
- 16: Haltemittel
- 17: Maskenwandung
- 18: Schlauch
- 19: Befestigungsmittel
- 20: Durchgangsöffnung
- 21: Schlauchaußenseite
- 22: Innenumfangsfläche
- 23: erste Durchgangshülse
- 24: Schraube
- 25: zweite Durchgangshülse
- 26: Mutter
- 27: Klemmhülse
- 28: Druckanschluss
- 29: Feststoff
- 30: Membran-Druckhülse
- 31: Membraninnenvolumen
- 32: Druckanschluss
- 33: Verbindungsmantel
- 34: Verbindungsgelenk
- 35: Maskenvolumen
- 36: Gasauslassabschnitt
- 37: Membran-Saughülse
- 38: Membraninnenvolumen
- 39: Federarm
- 40: Beatmungsgasleitungsrichtung
- 41: Ballondichtung
- 42: Ballonmantel
- 43: Gasöffnung
- 44: Ballonvolumen
- 45: Schlauchdichtung
- 46: Haltebandanordnung
- 47: Stirnband
- 48: Nackenband
- 49: Maskenrand
- 50: Silikonlippe
- 51: Randkammer
- 52: Randvolumen
- 53: Druckanschluss
- 54: Druckluftleitung
- 55: Haltering
- 56: Haken
- 57: Halteöse
- 58: Kopfband
- 60: Pumpe
- 70: Gasquelle
- 80: Blender
- 90: Befeuchter
- 100: Beatmungsgerät

## Patentansprüche

1. Maskenanordnung (10) für ein Beatmungsgerät (100) zum Beatmen eines Patienten (11), aufweisend eine Atemmaske (12) zum Positionieren über dem Mund (13) und über der Nase (14) des Patienten (11), eine einen Schlauch (18) aufweisende Nasenkanülenanordnung (15) zum Leiten eines Beatmungsgases in die Nase (14) des Patienten (11), und ein Haltemittel (16) zum Halten der Nasenkanülenanordnung (15) an der Atemmaske (12),
**dadurch gekennzeichnet, dass**
das Haltemittel (16) ein Befestigungsmittel (19) aufweist, das zwischen einem ersten Funktionszustand zum Fixieren des Schlauchs (18) in einer festen Halteposition an der Atemmaske (12) und einem zweiten Funktionszustand zum Freigeben des Schlauchs (18) aus der festen Halteposition, für eine Verschiebebewegung zwischen dem Schlauch (18) und der Atemmaske (12), verstellbar ist.

2. Maskenanordnung (10) nach Anspruch 1,
**dadurch gekennzeichnet, dass**
das Befestigungsmittel (19) an der Maskenwandung (17) eine erste Durchgangshülse (23) mit einem sich zum Patienten (11), der die Atemmaske (12) trägt, hin konisch verjüngenden Innengewinde und am Schlauch (18) eine mantelförmig um einen Teil des Schlauchs (18) herum angeordnete, Schraube (24) mit einem konischen Außengewinde zum Verschrauben mit dem konischen Innengewinde der ersten Durchgangshülse (23) und dadurch zum Fixieren des Schlauchs (18) in der Halteposition aufweist.

3. Maskenanordnung (10) nach Anspruch 1,
**dadurch gekennzeichnet, dass**
das Befestigungsmittel (19) an der Maskenwandung (17) eine mantelförmig um einen Teil des Schlauchs (18) herum ausgestaltete, elastisch verformbare zweite Durchgangshülse (25) mit einem Außengewinde und eine Mutter (26) mit einem konischen Innengewinde zum Verschrauben mit dem Außengewinde der zweiten Durchgangshülse (25) und dadurch zum Fixieren des Schlauchs (18) in der Halteposition aufweist.

4. Maskenanordnung (10) nach Anspruch 3,
**dadurch gekennzeichnet, dass**
in einer Radialrichtung betrachtet zwischen einer Schlauchaußenseite (21) und einer Innenumfangsfläche der zweiten Durchgangshülse (25) eine elastisch verformbare Klemmhülse (27) mantelförmig um einen Teil des Schlauchs (18) herum ausgestaltet ist, wobei das Material der Klemmhülse (27) eine höhere Festigkeit als das Material des Schlauchs (18) aufweist.

5. Maskenanordnung (10) nach einem der voranstehenden Ansprüche,
**dadurch gekennzeichnet, dass**
das Befestigungsmittel (19) eine an der Maskenwandung (17) angebrachte, elastisch verformbare Membran-Saughülse (37) mit einem Membraninnenvolumen (38) aufweist und die Membran-Saughülse (37) im Bereich der Durchgangsöffnung (20) mantelförmig um den Schlauch (18) herum ausgestaltet ist, wobei die Membran-Saughülse (37) einen Druckanschluss (28) zum Erzeugen eines Unterdrucks im Membraninnenvolumen (38) aufweist.

6. Maskenanordnung (10) nach Anspruch 5,
**dadurch gekennzeichnet, dass**
im Membraninnenvolumen (38) ein elastisch und/oder plastisch verformbarer Feststoff (29) angeordnet ist.

7. Maskenanordnung (10) nach einem der voranstehenden Ansprüche,
**dadurch gekennzeichnet, dass**
das Befestigungsmittel (19) im Bereich der Durchgangsöffnung (20) eine mantelförmig um den Schlauch (18) herum angebrachte, elastisch verformbare Membran-Druckhülse (30) mit einem Membraninnenvolumen (31) aufweist, wobei die Membran-Druckhülse (30) einen Druckanschluss (32) zum Erzeugen eines Überdrucks im Membraninnenvolumen (31) aufweist.

8. Maskenanordnung (10) nach einem der voranstehenden Ansprüche,
**dadurch gekennzeichnet, dass**
der Schlauch (18) im Bereich der Durchgangsöffnung (20) eine dünnere Schlauchwandung als in einem Bereich außerhalb der Durchgangsöffnung (20), eine elastisch verformbare Schlauchwandung und/oder eine Schlauchwandung mit einer höheren Elastizität als in einem Bereich außerhalb der Durchgangsöffnung (20) aufweist.

9. Maskenanordnung (10) nach einem der voranstehenden Ansprüche,
**dadurch gekennzeichnet, dass**
das Befestigungsmittel (19) einen mantelförmig um den Schlauch (18) herum ausgestalteten und im Bereich der Durchgangsöffnung (20) an der Maskenwandung (17) angebrachten, thermoplastisch verformbaren Verbindungsmantel (33) aufweist.

10. Maskenanordnung (10) nach einem der voranstehenden Ansprüche,
**gekennzeichnet durch**
ein Verbindungsgelenk (34) zum angelenkten Halten des Schlauchs (18) in der Durchgangsöffnung (20).

11. Maskenanordnung (10) nach einem der voranstehenden Ansprüche,
**dadurch gekennzeichnet, dass**
die Nasenkanülenanordnung (15) einen Gasauslassabschnitt (36) zum Auslassen des Beatmungsgases in die Nase (14) des Patienten (11) aufweist, wobei der Gasauslassabschnitt (36) düsenförmig ausgestaltet ist, sich konisch verjüngt oder wenigstens einen elastisch verformbaren Federarm (39), zum druckbeaufschlagten Halten des Gasauslassabschnitts (36) in der Nase (14), aufweist.

12. Maskenanordnung (10) nach einem der voranstehenden Ansprüche,
**dadurch gekennzeichnet, dass**
die Nasenkanülenanordnung (15) einen Gasauslassabschnitt (36) zum Auslassen des Beatmungsgases in die Nase (14) des Patienten (11) aufweist, wobei im und/oder am Gasauslassabschnitt (36) eine Ballondichtung (41) mit einem Ballonmantel (42) ausgestaltet ist, wobei der Ballonmantel (42) eine Gasöffnung (43) zum Zuführen des Beatmungsgases in der Nasenkanülenanordnung (15) bei einer Beatmung des Patienten (11) in ein durch den Ballonmantel (42) gebildetes Ballonvolumen (44) zum druckbeaufschlagten Halten des Gasauslassabschnitts (36) in der Nase (14) aufweist.

13. Maskenanordnung (10) nach einem der voranstehenden Ansprüche,
**dadurch gekennzeichnet, dass**
die Nasenkanülenanordnung (15) einen Gasauslassabschnitt (36) zum Auslassen des Beatmungsgases in die Nase (14) des Patienten (11) aufweist, wobei die Maskenanordnung (10) ferner eine aufblasbare Schlauchdichtung (45) aufweist, die ringförmig um den Gasauslassabschnitt (36) herum ausgestaltet ist.

## Claims

1. Mask assembly (10) for a ventilation apparatus (100) for ventilating a patient (11), having a respiratory mask (12) for positioning over the mouth (13) and over the nose (14) of the patient (11), a nasal cannula assembly (15), which has a tube (18), for guiding a ventilation gas into the nose (14) of the patient (11), and a holding means (16) for holding the nasal cannula assembly (15) on the respiratory mask (12), **characterized in that**
the holding means (16) has a fastening means (19) which is adjustable between a first functional state for fixing the tube (18) in a fixed holding position on the respiratory mask (12) and a second functional state for releasing the tube (18) from the fixed holding position, for a displacement movement between the tube (18) and the respiratory mask (12).

2. Mask assembly (10) according to Claim 1,
**characterized in that**
the fastening means (19) has, on the mask wall (17), a first through sleeve (23) having an internal thread tapering conically towards the patient (11) wearing the respiratory mask (12) and, on the tube (18), a screw (24) which is arranged in the form of a shell around part of the tube (18) and has a conical external thread for screwing together with the conical internal thread of the first through sleeve (23) and thus for fixing the tube (18) in the holding position.

3. Mask assembly (10) according to Claim 1,
**characterized in that**
the fastening means (19) has, on the mask wall (17), an elastically deformable second through sleeve (25) which is configured in the form of a shell around part of the tube (18) and has an external thread and a nut (26) having a conical internal thread for screwing together with the external thread of the second through sleeve (25) and thus for fixing the tube (18) in the holding position.

4. Mask assembly (10) according to Claim 3,
**characterized in that**
as viewed in a radial direction between a tube outer side (21) and an inner circumferential surface of the second through sleeve (25), an elastically deformable clamping sleeve (27) is configured in the form of a shell around part of the tube (18), the material of the clamping sleeve (27) being stronger than the material of the tube (18).

5. Mask assembly (10) according to one of the preceding claims,
**characterized in that**
the fastening means (19) has an elastically deformable membrane suction sleeve (37) which is attached to the mask wall (17) and has a membrane inner volume (38), and the membrane suction sleeve (37) in the region of the through opening (20) is configured in the form of a shell around the tube (18), the membrane suction sleeve (37) having a pressure connection (28) for generating a negative pressure in the membrane inner volume (38).

6. Mask assembly (10) according to Claim 5,
**characterized in that**
an elastically and/or plastically deformable solid material (29) is arranged in the membrane inner volume (38) .

7. Mask assembly (10) according to one of the preceding claims,
**characterized in that**
the fastening means (19) in the region of the through opening (20) has an elastically deformable membrane compression sleeve (30) which is mounted in the form of a shell around the tube (18) and has a membrane inner volume (31), the membrane compression sleeve (30) having a pressure connection (32) for generating a positive pressure in the membrane inner volume (31).

8. Mask assembly (10) according to one of the preceding claims,
**characterized in that**
the tube (18) in the region of the through opening (20) has a thinner tube wall than in a region outside the through opening (20), an elastically deformable tube wall and/or a tube wall of higher elasticity than in a region outside the through opening (20).

9. Mask assembly (10) according to one of the preceding claims,
**characterized in that**
the fastening means (19) has a thermoplastically deformable connecting shell (33) which is configured in the form of a shell around the tube (18) and is attached to the mask wall (17) in the region of the through opening (20) .

10. Mask assembly (10) according to one of the preceding claims,
**characterized by**
a connecting joint (34) for the articulated holding of the tube (18) in the through opening (20).

11. Mask assembly (10) according to one of the preceding claims,
**characterized in that**
the nasal cannula assembly (15) has a gas outlet portion (36) for letting out the ventilation gas into the nose (14) of the patient (11), the gas outlet portion (36) being configured in the form of a nozzle, and tapering conically or having at least one elastically deformable spring arm (39), for the pressurized holding of the gas outlet portion (36) in the nose (14).

12. Mask assembly (10) according to one of the preceding claims,
**characterized in that**
the nasal cannula assembly (15) has a gas outlet portion (36) for letting out the ventilation gas into the nose (14) of the patient (11), a balloon seal (41) with a balloon shell (42) being formed in and/or on the gas outlet portion (36), the balloon shell (42) having a gas opening (43) for feeding the ventilation gas in the nasal cannula assembly (15) during respiration of the patient (11) into a balloon volume (44), which is formed by the balloon shell (42), for the pressurized holding of the gas outlet portion (36) in the nose (14).

13. Mask assembly (10) according to one of the preceding claims,
**characterized in that**
the nasal cannula assembly (15) has a gas outlet portion (36) for letting out the ventilation gas into the nose (14) of the patient (11), the mask assembly (10) furthermore having an inflatable tube seal (45) which is formed annularly around the gas outlet portion (36).

## Revendications

1. Agencement (10) de masque destiné à un respirateur (100) dévolu à la ventilation d'un patient (11), comprenant un masque respiratoire (12) conçu pour être mis en place sur la bouche (13) et, sur le nez (14) dudit patient (11), un dispositif à canule nasale (15) muni d'un tuyau souple (18) et conçu pour guider un gaz respiratoire vers ledit nez (14) du patient (11), et un moyen de retenue (16) conçu pour maintenir ledit dispositif à canule nasale (15) sur ledit masque respiratoire (12),
**caractérisé par le fait que**
le moyen de retenue (16) est doté d'un moyen de fixation (19) pouvant être réglé entre un premier état fonctionnel affecté au blocage à demeure du tuyau souple (18) sur le masque respiratoire (12), en un emplacement fixe de maintien, et un second état fonctionnel affecté à la libération dudit tuyau souple (18), à partir dudit emplacement fixe de maintien, en vue d'un mouvement de coulissement s'opérant entre ledit tuyau souple (18) et ledit masque respiratoire (12).

2. Agencement (10) de masque, selon la revendication 1,
**caractérisé par le fait que**
le moyen de fixation (19) comprend, sur la paroi (17) du masque, une première douille de passage (23) dotée d'un filetage intérieur s'amenuisant tronconiquement en direction du patient (11) porteur dudit masque respiratoire (12) et, sur le tuyau souple (18), une vis (24) ceinturant, en forme d'enveloppe, une partie dudit tuyau souple (18) et pourvue d'un filetage extérieur tronconique dédié à la solidarisation vissée avec le filetage intérieur tronconique de ladite une première douille de passage (23) et, de ce fait, au blocage à demeure dudit tuyau souple (18) à l'emplacement de maintien.

3. Agencement (10) de masque, selon la revendication 1,
**caractérisé par le fait que**
le moyen de fixation (19) comprend une seconde douille de passage (25) élastiquement déformable, située sur la paroi (17) du masque, munie d'un filetage extérieur et configurée pour ceinturer, en forme d'enveloppe, une partie du tuyau souple (18), et un écrou (26) nanti d'un filetage intérieur tronconique dédié à la solidarisation vissée avec le filetage extérieur de ladite seconde douille de passage (25) et, de ce fait, au blocage à demeure dudit tuyau souple (18) à l'emplacement de maintien.

4. Agencement (10) de masque, selon la revendication 3,
**caractérisé par le fait**
**qu'**en observant dans une direction radiale, une douille de serrage (27) élastiquement déformable est configurée pour ceinturer, en forme d'enveloppe, une partie du tuyau souple (18) entre une face extérieure (21) dudit tuyau souple et une surface du pourtour intérieur de la seconde douille de passage (25), le matériau de ladite douille de serrage (27) étant doué d'une solidité supérieure à celle du matériau dudit tuyau souple (18).

5. Agencement (10) de masque, selon l'une des revendications précédentes,
**caractérisé par le fait que**
le moyen de fixation (19) est muni d'une douille d'aspiration (37) à membrane, implantée sur la paroi (17) du masque, élastiquement déformable, et dont la membrane présente un volume intérieur (38), laquelle douille d'aspiration (37) à membrane est configurée pour ceinturer, en forme d'enveloppe, le tuyau souple (18) dans la région de l'orifice traversant (20), ladite douille d'aspiration (37) à membrane étant dotée d'un raccord de pression (28) en vue d'engendrer une dépression dans ledit volume intérieur (38) de la membrane.

6. Agencement (10) de masque, selon la revendication 5,
**caractérisé par le fait**
**qu'**une substance solide (29), élastiquement et/ou plastiquement déformable, est disposée dans le volume intérieur (38) de la membrane.

7. Agencement (10) de masque, selon l'une des revendications précédentes,
**caractérisé par le fait que**
le moyen de fixation (19) est muni, dans la région de l'orifice traversant (20), d'une douille de pression (30) à membrane, élastiquement déformable, implantée en forme d'enveloppe autour du tuyau souple (18), et dont la membrane présente un volume intérieur (31), ladite douille de pression (30) à membrane étant nantie d'un raccord de pression (32) en vue d'engendrer une surpression dans ledit volume intérieur (31) de la membrane.

8. Agencement (10) de masque, selon l'une des revendications précédentes,
**caractérisé par le fait que**
le tuyau souple (18) présente, dans la région de l'orifice traversant (20), une paroi plus mince que dans une région située à l'extérieur dudit orifice traversant (20), une paroi élastiquement déformable et/ou une paroi douée d'une plus grande élasticité que dans une région située à l'extérieur dudit orifice traversant (20).

9. Agencement (10) de masque, selon l'une des revendications précédentes,
**caractérisé par le fait que**
le moyen de fixation (19) est pourvu d'une gaine de jonction (33) thermoplastiquement déformable, configurée pour ceinturer le tuyau souple (18), en forme d'enveloppe, et implantée sur la paroi (17) du masque dans la région de l'orifice traversant (20).

10. Agencement (10) de masque, selon l'une des revendications précédentes,
**caractérisé par**
une articulation de liaison (34) conçue pour maintenir, de manière articulée, le tuyau souple (18) dans l'orifice traversant (20).

11. Agencement (10) de masque, selon l'une des revendications précédentes,
**caractérisé par le fait que**
le dispositif à canule nasale (15) comporte une zone (36) de sortie de gaz, conçue pour admettre le gaz respiratoire dans le nez (14) du patient (11), sachant que ladite zone (36) de sortie de gaz est réalisée en forme de tuyère, s'amenuise tronconiquement, ou est dotée d'au moins un bras (39) élastiquement déformable pour maintenir ladite zone (36) de sortie de gaz dans le nez (14), avec sollicitation par une pression.

12. Agencement (10) de masque, selon l'une des revendications précédentes,
**caractérisé par le fait que**
le dispositif à canule nasale (15) comporte une zone (36) de sortie de gaz, conçue pour admettre le gaz respiratoire dans le nez (14) du patient (11), sachant qu'une garniture d'étanchement (41) à ballonnet, pourvue d'une enveloppe (42), est ménagée dans et/ou sur ladite zone (36) de sortie de gaz, l'enveloppe (42) dudit ballonnet présentant une ouverture (43) dédiée au gaz en vue de délivrer, dans un volume (44) du ballonnet formé par ladite enveloppe (42) dudit ballonnet, le gaz respiratoire présent dans ledit dispositif à canule nasale (15) au cours d'une ventilation du patient (11), afin de maintenir ladite zone (36) de sortie de gaz dans le nez (14), avec sollicitation par une pression.

13. Agencement (10) de masque, selon l'une des revendications précédentes,
**caractérisé par le fait que**
le dispositif à canule nasale (15) comporte une zone (36) de sortie de gaz, conçue pour admettre le gaz respiratoire dans le nez (14) du patient (11), sachant que ledit agencement (10) de masque est pourvu, par ailleurs, d'une garniture d'étanchement gonflable (45) à tuyau souple, configurée pour ceinturer annulairement ladite zone (36) de sortie de gaz.
